# EUROPEAN PATENT APPLICATION

(11) **EP 4 023 287 A1**
(43) Date of publication of application: **06.07.2022**
(21) Application number: 19943649.4
(22) Date of filing: 27.08.2019
(51) Int. Cl.: A61N 5/06, A61B 10/00

(54) **TREATMENT SUPPORT DEVICE AND THERAPEUTIC LIGHT CONTROL METHOD**

(71) Applicant: Shimadzu Corporation, Kyoto-shi Kyoto 604-8511 (JP)
(72) Inventor: TSUMATORI, Hiroyuki, Kyoto-shi, Kyoto 604-8511 (JP)
(74) Representative: Müller-Boré & Partner Patentanwälte PartG mbB
(86) International application number: PCT/JP2019/033560
(87) International publication number: WO 2021/038726

(57) **Abstract**

A treatment support device (1) configured to control therapeutic light irradiated toward a treatment site of a subject (ST) to whom a therapeutic agent containing a fluorescent dye has been administered and excite the fluorescent dye by means of the therapeutic light to perform treatment is provided with: a light source (242) for emitting the therapeutic light; a control unit (17) for controlling the irradiation time and the irradiation intensity of the therapeutic light; and a detection unit (182, 17) for detecting the intensity of the fluorescence generated from the fluorescent dye, when the therapeutic light is being emitted. The control unit (17) controls at least one of the irradiation time and the irradiation intensity of the therapeutic light based on the irradiation intensity of the fluorescence.

## Description

### Technical Field

The present invention relates to a treatment support device and a therapeutic light control method.

### Background of the Invention

In recent years, photoimmunotherapy has attracted attention as one of cancer treatment methods. In this photoimmunotherapy, a therapeutic agent (RM-1929) in which a fluorescent reagent (IR700) and an antibody of an epidermal growth factor receptor (EGFR) are bound is injected into a subject. When the therapeutic agent is injected into the subject, the antibody binds to the surface of cancer cells. In this state, when a subject is irradiated with near-infrared light (therapeutic light) having a wavelength of about 600 nm to about 750 nm for a predetermined period, heat is generated in the therapeutic agent (RM-1929), thereby destroying the cancer cells and detaching the ligand portion, which is a hydrophilic group of IR700, which in turn forms an aggregate and quenches (see, Non-Patent Documents 1 and 2).

In such photoimmunotherapy, the irradiation time of the therapeutic light is determined in advance based on the treatment site and the light intensity of the therapeutic light, and the therapeutic light is emitted to the treatment site for the irradiation time.

### Prior Art Document

### Non-Patent Document

**Non Patent Document 1:** K. Sato et al., "Photo-induced ligand release from a silicon phthalocyanine dye conjugated with monoclonal antibodies; A mechanism of cancer cell cytotoxicity after near infrared photoimmunotherapy," ACS Central Science, 7 November 2018
**Non Patent Document 2:** Shimadzu Corp., First Success in Developing Optical Remote Controller Switches that Kill Only Target Cells - Expected to Contribute to Cancer Treatment with Less Side Effects, [online], [Search on March 10, 2019], Internet <URL: https://WWW.SHIMADZU.CO.JP/news/press/9qbnqud1mddlrfby.html>

### SUMMARY OF THE INVENTION

### Problems to be Solved by the Invention

However, in such photoimmunotherapy, the treatment effect (i.e., the progress of treatment) may differ depending on a subject. When therapeutic light is emitted for a predetermined time, in some cases, the therapeutic light may be continuously emitted even though the treatment has already been completed. In this case, the burden on the subject increases. On the other hand, in some cases, the irradiation of the therapeutic light may be stopped even though the treatment has not been completed. In this case, there is a need to perform the treatment again.

Further, in such photoimmunotherapy, since the burden on the subject increases when the intensity of the therapeutic light is high, there is a demand for lowering the irradiation intensity of the therapeutic light as much as possible.

The present invention has been made in view of such circumstances. It is an object of the present invention to provide a treatment support device and a therapeutic light control method capable of appropriately controlling therapeutic light in accordance with a treatment effect (that is, the progress of treatment).

### Means for Solving the Problem

According to a first aspect of the present invention, a treatment support device configured to control therapeutic light emitted toward a treatment site of a subject to whom a therapeutic agent containing a fluorescent dye has been administered and excite the fluorescent dye by means of the therapeutic light to perform treatment, is provided with:
a light source configured to emit the therapeutic light;
a control unit configured to control an irradiation time and irradiation intensity of the therapeutic light; and
a detection unit configured to detect intensity of fluorescence generated from the fluorescent dye, when the therapeutic light is being emitted,
wherein the control unit controls at least one of the irradiation time and the irradiation intensity of the therapeutic light based on the intensity of the fluorescence.

According to a second aspect of the present invention, a therapeutic light control method configured to control therapeutic light emitted toward a treatment site of a subject to whom a therapeutic agent containing a fluorescent dye has been administered and excite the fluorescent dye by means of the therapeutic light to perform treatment, includes the steps of:
emitting the therapeutic light;
controlling an irradiation time and irradiation intensity of the therapeutic light; and
detecting intensity of the fluorescence generated from the fluorescent dye, when the therapeutic light is being emitted,
wherein the step of controlling the therapeutic light controls at least one of the irradiation time and the irradiation intensity of the therapeutic light based on the intensity of the fluorescence.

### Effects of the Invention

According to the present invention, the treatment support device and the therapeutic light control method capable of appropriately controlling therapeutic light in accordance with a treatment effect (i.e., the progress of treatment) can be realized.

### BRIEF DESCRIPTION OF THE DRAWINGS

FIG. 1 is a perspective view showing an embodiment of a treatment support device of the present invention.
FIG. 2 is a side view of the treatment support device shown in FIG. 1.
FIG. 3 is a plan view of the treatment support device shown in FIG. 1.
FIG. 4 is a block diagram showing a main control system of the treatment support device shown in FIG. 1.
FIG. 5 is a flowchart of a therapeutic light control program executed by the treatment support device shown in FIG. 1.
FIG. 6 shows an example of an image displayed on an image display unit provided on the treatment support device shown in FIG. 1.
FIG. 7 is a graph showing a relationship between a therapeutic light irradiation time t and fluorescence strength P of the treatment support device shown in FIG. 1.
FIG. 8 is a flowchart of a therapeutic light control program according to Modification 1 of the present invention.

### EMBODIMENTS FOR CARRYING OUT THE INVENTION

Hereinafter, a treatment support device and a therapeutic light control method according to the present invention will be described in detail based on suitable embodiments shown in the accompanying drawings.

### <Embodiment>

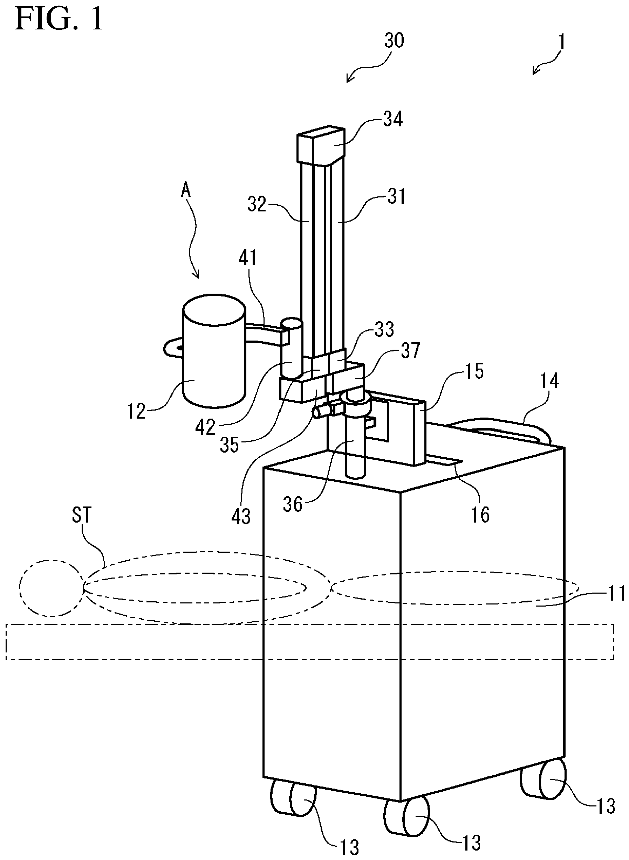

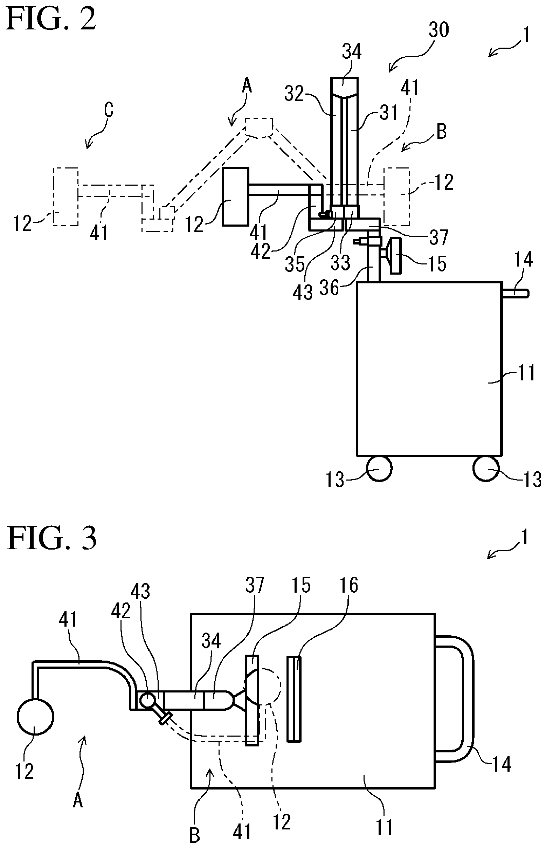

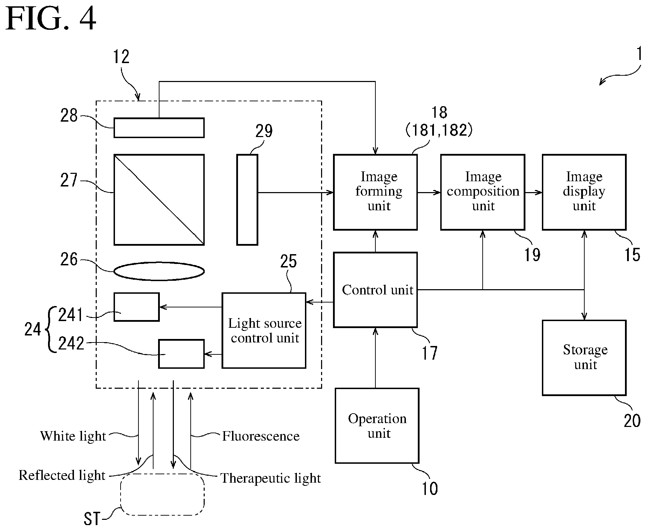

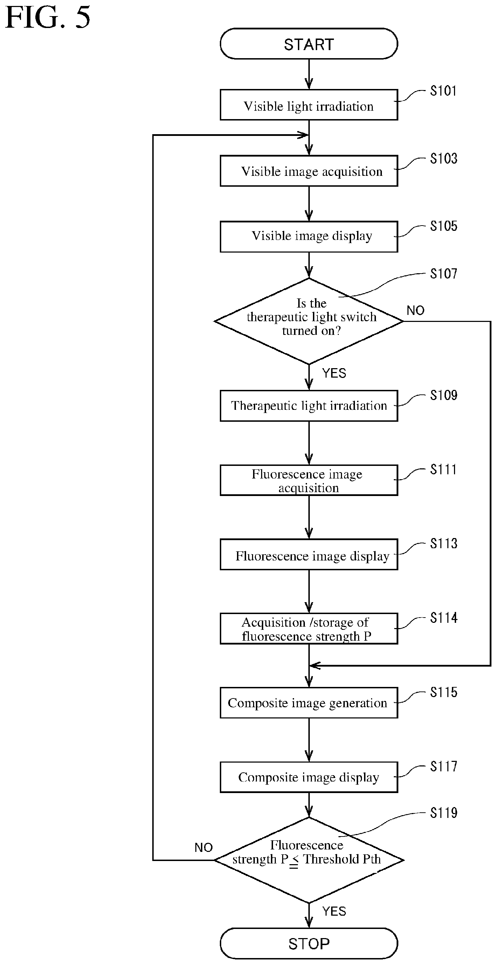

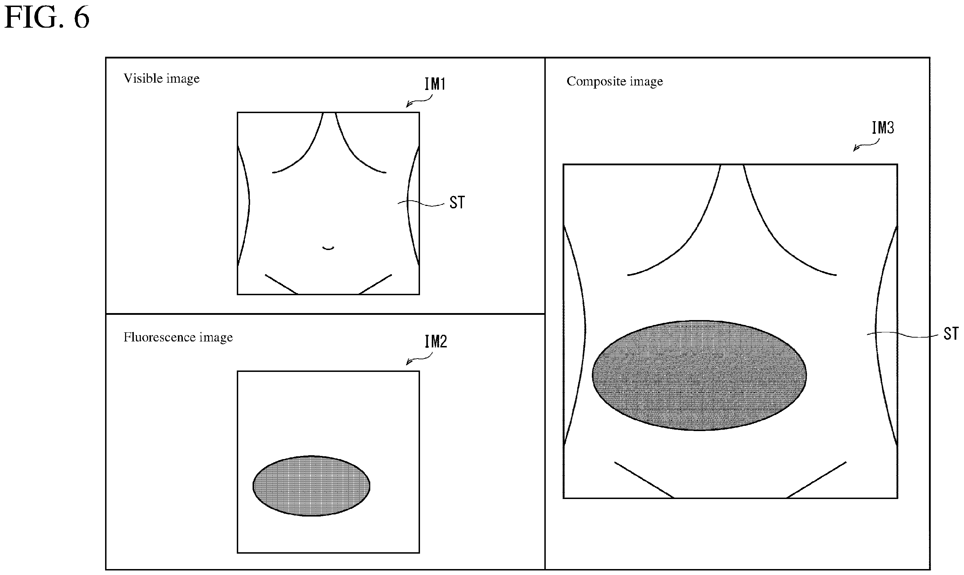

FIG. 1 is a perspective view showing an embodiment of a treatment support device of the present invention. FIG. 2 is a side view of the treatment support device shown in FIG. 1. FIG. 3 is a plan view of the treatment support device shown in FIG. 1. FIG. 4 is a block diagram showing a main control system of the treatment support device shown in FIG. 1. FIG. 5 is a flowchart of a therapeutic light control program executed by the treatment support device shown in FIG. 1. FIG. 6 shows an example of an image displayed on an image display unit provided on the treatment support device shown in FIG. 1. Note that in the following description, for convenience of description, the upper side in FIGS. 1, 2, and 6 is referred to as "upper (upper side)," and the lower side is referred to as "lower (lower side)".

The treatment support device 1 shown in FIG. 1 is a device for emitting excitation light (hereinafter also referred to as "therapeutic light") to a therapeutic agent (RM-1929) which has been injected into the body of the subject ST in which a fluorescent reagent (IR700) and an antibody of an epidermal growth factor receptor (EGFR) is bound to image the fluorescence emitted form the fluorescent reagent (IR700). By using the treatment support device 1, as will be described later, when performing photoimmunotherapy on the subject ST, it is possible to confirm, for example, the treatment position of the subject ST and the treatment effect (that is, the progress of treatment) and control the irradiation time and the irradiation intensity of the therapeutic light.

The treatment support device 1 is provided with a carriage 11 having four wheels 13, an arm mechanism 30 mounted on the front of the carriage 11 in the traveling direction (in the left direction in FIGS. 2 and 3) on the upper surface of the carriage 11, a lighting and imaging unit 12 provided on the arm mechanism 30 via the sub-arm 41, and an image display unit 15 as a monitor. At the rear portion of the carriage 11 in the traveling direction, a handle 14 for moving the carriage 11 is attached. A recess 16 for attaching a remote control for remotely operating the treatment support device 1 is formed on the upper surface of the carriage 11.

Further, as will be described in detail later, when the power is applied to the treatment support device 1, a therapeutic light control program is executed (that is, each Step shown in FIG. 5 is executed), a visible image IM1, a fluorescence image IM2, and a composite image IM3 are displayed on the image display unit 15, and the irradiation time and the irradiation intensity of the therapeutic light are controlled (see FIG. 6).

The above-described arm mechanism 30 is provided on the front side of the carriage 11 in the traveling direction. The arm mechanism 30 is provided with a first arm member 31 connected, via a hinge portion 33, to a support portion 37 provided on a support 36 erected on the front side of the carriage 11 in the traveling direction. The first arm member 31 is swingable with respect to the carriage 11 via the support 36 and the support portion 37 by the action of the hinge portion 33. Note that the above-described image display unit 15 is attached to the support 36.

At the upper end of the first arm member 31, a second arm member 32 is connected by the hinge portion 34. The second arm member 32 is swingable with respect to the first arm member 31 by the action of the hinge portion 34. Therefore, the first arm member 31 and the second arm member 32 can take an imaging posture as shown by the virtual line labelled C in FIG. 2 and a standby posture as shown by the solid line labeled A in FIGS. 1 to 3. In the imaging posture, the first arm member 31 and the second arm member 32 are opened at a predetermined angle about the hinge portion 34 which is a connecting portion between the first arm member 31 and the second arm member 32. In the standby posture, the first arm member 31 and the second arm member 32 are closely arranged.

At the lower end of the second arm member 32, a support portion 43 is connected by a hinge portion 35. The support portion 43 is swingable with respect to the second arm member 32 by the action of the hinge portion 35. A rotation shaft 42 is supported by the support portion 43. The sub-arm 41 supporting the lighting and imaging unit 12 is rotatable about the rotation shaft 42 provided at the tip end of the second arm member 32. For this reason, the lighting and imaging unit 12 is moved by this rotation of the sub-arm 41 between a position on the front side of the carriage 11 in the traveling direction with respect to the arm mechanism 30 for taking the imaging posture or the standby posture as indicated by the solid line labeled A in FIGS. 1 to 3 or as indicated by the virtual line labeled C in FIG. 2 and a position on the rear side of the carriage 11 in the traveling direction with respect to the arm mechanism 30 for taking a posture for moving the carriage 11 as indicated by the virtual line labeled B in FIGS. 2 and 3.

As shown in FIG. 4, the lighting and imaging unit 12 is provided with a light source unit 24, a light source control unit 25, a zoom lens 26, a prism 27, a white light sensor 28, and an excitation light sensor 29. When the treatment support device 1 is used, the lighting and imaging unit 12 is preferably set so as to be separated from the affected area of the subject ST by about several tens of centimeters.

The light source unit 24 is provided with a first light source 241, which is a white light source, and a second light source 242, which is an excitation light source. When the first light source 241 is turned on, white light is emitted toward the subject ST. The light of the white light is reflected by the subject ST, and the reflected white light is detected by the white light sensor 28.

The second light source 242 emits excitation light (therapeutic light) to excite the fluorescent reagent (IR700). When the second light source 242 is turned on, near-infrared light (excitation light) having a wavelength of 600 nm to 750 nm is emitted toward the subject ST, and the fluorescent reagent (IR700) of the therapeutic agent (RM-1929) which has been administered to the subject ST is excited. When the fluorescent reagent (IR700) is excited, the near-infrared light having a peak of about 700 nm is emitted as fluorescence and detected by the excitation light sensor 29.

The light source control unit 25 has a function of controlling the lighting of the first light source 241. With this function, it is possible to cause the first light source 241 to start emission of white light and stop emission of the white light. Further, the light source control unit 25 has a function of controlling the lighting of the second light source 242, and a function of controlling the amount of light of the second light source 242. These functions control the irradiation time and the irradiation intensity of the therapeutic light emitted from the second light source 242. The light source control unit 25 is connected to the control unit 17 for collectively controlling the treatment support device 1, and the first light source 241 and the second light source 242 are controlled in accordance with the instruction from the control unit 17.

The reflected light (white light) reflected by the subject ST and the fluorescence generated by the fluorescent reagent (IR700) in the subject ST are incident on the zoom lens 26. With the zoom lens 26, the reflected light (white light) is focused on the white light sensor 28, and the fluorescence is focused on the excitation light sensor 29. The light from the zoom lens 26, i.e., the white light and the fluorescence, is incident on the prism 27. The prism 27 is configured such that the white light and the fluorescence incident on the prism 27 are separated and that the white light is directed to the white light sensor 28 and the fluorescence is directed to the excitation light sensor 29.

The white light sensor 28 is an image sensor for detecting a part of the reflected light (white light) separated by the prism 27, and captures, for example, a visible image of the subject ST at the frame rate (30 frames/sec (60 fields/sec)) of the NTSC (National Television System Committee). The excitation light sensor 29 is an image sensor for detecting a part of the near-infrared light (fluorescence) separated by the prism 27, and captures the fluorescence image of the subject ST at the frame rate (e.g., 30 frames/sec (60 fields/sec)) of the NTSC.

Further, as shown in FIG. 4, the treatment support device 1 is provided with the control unit 17, an image forming unit 18, an image composition unit 19, a storage unit 20, and an operation unit 10. These units are arranged on the carriage 11.

The control unit 17 is composed of a CPU for executing a logical operation, a ROM for storing an operation program necessary for controlling the device, a RAM for temporarily storing data, etc., at the time of control, and has a function for controlling the entire device. The control unit 17 is electrically connected to the light source control unit 25, the image forming unit 18, the image composition unit 19, the image display unit 15, the storage unit 20, and the operation unit 10. When the power of the treatment support device 1 is turned on, the control unit 17 reads out the therapeutic light control program stored in the storage unit 20 and controls the above-described units.

The reflected light (white light) detected by the white light sensor 28 and the near-infrared light (fluorescence) detected by the excitation light sensor 29 are inputted to the image forming unit 18. Then, the image forming unit 18 form a visible image IM1 of 24 bits (= 3 × 8) composed of three colors of RGB (red, green, blue) by the reflected light (white light) detected by the white light sensor 28. Further, the image forming unit 18 forms a fluorescence image IM2 of 8 bits by the near-infrared light (fluorescence) detected by the excitation light sensor 29. In this embodiment, the image forming unit 18 functions as a first imaging unit 181 for acquiring the visible image IM1 by imaging the subject ST irradiated with the white light at the frame rate of the NTSC and a second imaging unit 182 for acquiring the fluorescence image IM2 by imaging the fluorescence generated by the fluorescent reagent (IR700) at the frame rate of the NTSC.

The image composition unit 19 composes the visible image IM1 and the fluorescence image IM2 formed by the image forming unit 18 to form (generate) a composite image IM3. As shown in FIG. 6, in this embodiment, the visible image IM1, the fluorescence image IM2, and the composite image IM3 are collectively displayed on the image display unit 15. By observing the composite image IM3, the physician can accurately grasp the treatment position of the subject ST and the treatment effect (i.e., the progress of treatment).

The storage unit 20 is configured to store a therapeutic light control program executed by the control unit 17, the luminance value, etc., of the fluorescence image IM2 formed by the image forming unit 18.

The operation unit 10 is a user interface for operating the treatment support device 1. For example, the operation unit 10 is configured to be able to emit the light from the light source unit 24, stop the emission of the light, adjust the brightness and the sensitivity, and set the display method, etc., of the image displayed on the image display unit 15.

Next, referring to FIG. 5, the therapeutic light control program executed by the control unit 17 will be described. The therapeutic light control program is read from the storage unit 20 and executed by the control unit 17, when the power of the treatment support device 1 is turned on.

As shown in FIG. 5, when the therapeutic light control program is executed, the control unit 17 controls the light source control unit 25 to turn on the first light source 241. When the first light source 241 is turned on, white light is emitted toward the subject ST. Upon completion of the processing of Step S101, the processing proceeds to Step S103.

In Step S103, the control unit 17 controls the first imaging unit 181 of the image forming unit 18 and acquires the visible image IM1 from the data of the white light sensor 28 inputted at the frame rate of the NTSC to the image forming unit 18. Upon completion of the processing of Step S103, the processing proceeds to Step S105.

In Step S105, the control unit 17 controls the image composition unit 19 and the image display unit 15, sends the visible image IM1 acquired in Step S103 to the image composition unit 19, and displays the visible image IM1 on the image display unit 15. Upon completion of the processing of Step S105, the processing proceeds to Step S107.

In Step S107, the control unit 17 determines whether or not the therapeutic light switch (switch for emitting the therapeutic light) of the operation unit 10 is turned on. When the therapeutic light switch is turned on (Step S107: YES), the processing proceeds to Step S109, and when the excitation light switch is turned off (Step S107: NO), the processing proceeds to Step S115.

In Step S109, the control unit 17 controls the light source control unit 25 to turn on the second light source 242. When the second light source 242 is turned on, the therapeutic light is emitted toward the subject ST. Upon completion of the processing of Step S109, the processing proceeds to Step S111.

In Step S111, the control unit 17 controls the second imaging unit 182 of the image forming unit 18 to acquire the fluorescence image IM2 from the data of the excitation light sensor 29 inputted to the image forming unit 18 at the frame rate of the NTSC. More specifically, in this embodiment, one frame of the fluorescence image IM2 is acquired and stored in the storage unit 20. Upon completion of the processing of Step S111, the processing proceeds to Step S113.

In Step S113, the control unit 17 controls the image composition unit 19 and the image display unit 15 to send the fluorescence image IM2 acquired and stored in Step S111 to the image composition unit 19 and causes the image display unit 15 to display the fluorescence image IM2. Upon completion of the processing of Step S113, the processing proceeds to Step S114.

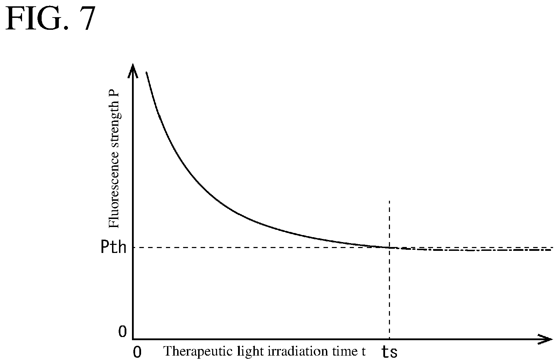

In Step S114, the control unit 17 obtains the irradiation intensity (hereinafter referred to as "fluorescence strength P") of the fluorescence from the fluorescence image IM2 acquired and stored in Step S111 and saves (memorizes) it in the storage unit 20. More specifically, in this embodiment, the control unit 17 identifies the pixel receiving the fluorescence in the fluorescence image IM2, obtains the fluorescence strength P (mW) based on the mean luminance value of the pixel, and stores the fluorescence strength P (mW) in the storage unit 20. FIG. 7 is a graph showing the relationship between the therapeutic light irradiation time t and the fluorescence strength P. As shown in FIG. 7, when the therapeutic light is emitted toward the subject ST in Step S109, fluorescence derived from the fluorescent reagent (IR700) in the subject ST is detected. Since the reaction of the fluorescent reagent (IR700) proceeds over time (i.e., the therapeutic light irradiation time t increases), the fluorescence strength P gradually decreases. It is known that when the fluorescence strength P becomes smaller than a predetermined threshold Pth, the amount of change in the fluorescence strength P becomes extremely small. Therefore, in this embodiment, when the fluorescence strength P has become equal to or lower than the predetermined threshold Pth (at the time ts in FIG. 7), it is configured to determine that the treatment has been completed (Step S119). In Step S114, the fluorescence strength P of each fluorescence image IM2 is obtained prior to Step S119. Upon completion of the processing of Step S114, the processing proceeds to Step S115.

In Step S115, the control unit 17 controls the image composition unit 19 to superimpose and compose the visible image IM1 acquired in Step S103 and the fluorescence image IM2 acquired in Step S111 to thereby generate a composite image IM3. Upon completion of the processing of Step S115, the processing proceeds to Step S117.

In Step S117, the control unit 17 controls the image display unit 15 to cause the image display unit 15 to display the composite image IM3 generated in Step S115. Upon completion of the processing of Step S117, the processing proceeds to Step S119.

In Step S119, the control unit 17 determines whether or not the fluorescence strength P stored in the storage unit 20 in Step S114 has become equal to or less than the predetermined threshold Pth. When the fluorescence strength P has become equal to or less than the predetermined threshold Pth, the control unit 17 determines that the treatment has been completed and ends the therapeutic light control program (Step S119: YES), and when the fluorescence strength P has not become equal to or less than the predetermined threshold Pth (Step S119: NO), the processing returns to Step S103 to repeat Steps S103 to S119.

As described above, when the composite image IM3 is obtained by the treatment support device 1 of this embodiment (i.e., when the therapeutic light control program is executed), the physician can accurately grasp the treatment position of the subject ST, the treatment effect (i.e., the progress of treatment), by observing the composite image IM3. Further, in this embodiment, the fluorescence strength P of each fluorescence image IM2 is obtained in Step S114. It is determined in Step S119 whether or not the fluorescence strength P has become equal to or less than the predetermined threshold Pth. When the fluorescence strength P has become equal to or less than the predetermined threshold Pth, it is determined that the treatment has been completed and the therapeutic light control program is ended (i.e., the therapeutic light irradiation is stopped). Therefore, unnecessary therapeutic light will not be emitted to the subject ST. In other words, the therapeutic light is appropriately controlled in accordance with the treatment effect (i.e., the progress of treatment).

Although the embodiment of the present invention has been described above, the present invention is not limited to the configuration of the embodiment described above, and various modifications can be made within the scope of the technical concept.

For example, in this embodiment, it is configured such that the fluorescence strength P is obtained based on the mean luminance value of the pixel receiving fluorescence in the fluorescence image IM2, but the present invention is not limited to such a configuration. For example, the fluorescence strength P may be obtained based on the maximum luminance value in the fluorescence image IM2.

Further, in the therapeutic light control program of this embodiment, it is configured such that when the fluorescence strength P has not become equal to or less than the predetermined threshold Pth (Step S119: NO), Steps S103 to S119 are repeated, and therapeutic light is emitted until the fluorescence strength P has become equal to or less than the predetermined threshold Pth (i.e., it is configured to control the irradiation time of the therapeutic light). However, the present invention is not limited to this configuration. For example, in a case where the fluorescence strength P has not yet become equal to or lower than the predetermined threshold Pth (Step S119: NO), the irradiation intensity of the therapeutic light may be increased. As described above, when the irradiation intensity of the therapeutic light is increased, the reaction of the fluorescent reagent (IR700) proceeds, so that the irradiation time of the therapeutic light (that is, the treatment time) can be shortened. Thus, in this embodiment, at least one of the irradiation time and the irradiation intensity of the therapeutic light may be controlled.

### <Modification 1>

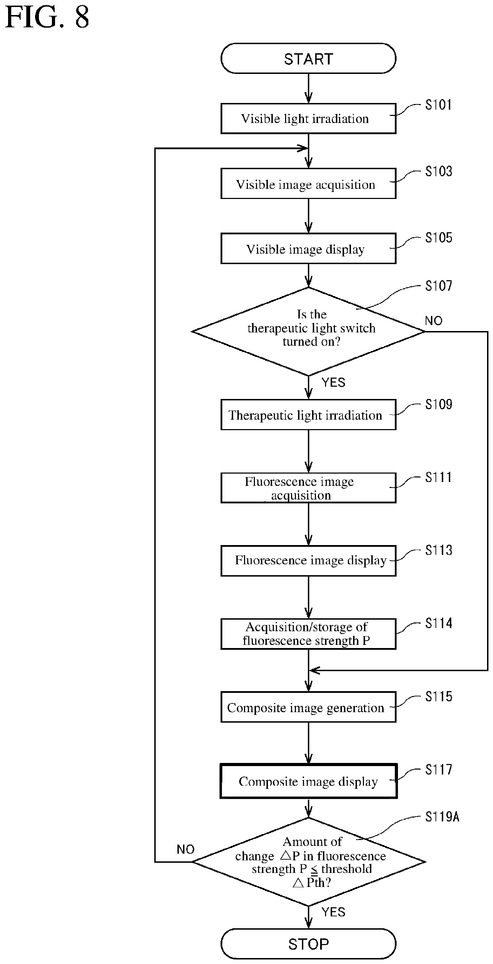

FIG. 8 is a flowchart of a therapeutic light control program shown as Modification 1 of the embodiment. As shown in FIG. 8, in this modification, it differs from the embodiment in that in Step S119A, the control unit 17 obtains the amount of change ΔP in the fluorescence strength from the difference between the fluorescence strength P₍ₙ₎ stored in the storage unit 20 in Step S114 and the fluorescence strength P₍ₙ₋₁₎ stored in the storage unit 20 in Step S114 one frame earlier, and determines whether or not the amount of change ΔP has become equal to or less than the predetermined threshold ΔPth. When the amount of change ΔP in the fluorescence strength P has become equal to or less than the predetermined threshold ΔPth, the control unit 17 determines that the treatment has been completed and ends the therapeutic light control program (Step S I 19A: YES). On the other hand, when the amount of change ΔP in the fluorescence strength P has not yet become equal to or less than the predetermined threshold ΔPth (Step S119A: NO), the processing returns to Step S 103 to repeat Steps S103 to S119.

In this manner, in this modification, attention is paid to the amount of change ΔP in the fluorescence strength P, and when the amount of change ΔP in the fluorescence strength P has become equal to or less than the predetermined threshold APth (that is, when the amount of change ΔP has become small), it is determined that the treatment has been completed, and the therapeutic light control program is finished (that is, the illumination of therapeutic light is stopped). Therefore, unnecessary therapeutic light will not be emitted to the subject ST. In other words, therapeutic light is appropriately controlled in accordance with the treatment effect (i.e., the progress of treatment).

It should be noted that the embodiments disclosed herein are illustrative in all respects and should not be considered limiting. The scope of the present invention is indicated by claims and not by the above-described descriptions, and is intended to include all modifications within the meanings and scopes equivalent to the claims.

### (Item 1)

A treatment support device configured to control therapeutic light emitted toward a treatment site of a subject to whom a therapeutic agent containing a fluorescent dye has been administered and excite the fluorescent dye by means of the therapeutic light to perform treatment, the treatment support device comprising:
a light source configured to emit the therapeutic light;
a control unit configured to control an irradiation time and irradiation intensity of the therapeutic light; and
a detection unit configured to detect intensity of fluorescence generated from the fluorescent dye, when the therapeutic light is being emitted,
wherein the control unit controls at least one of the irradiation time and the irradiation intensity of the therapeutic light based on the intensity of the fluorescence.

According to the treatment support device described in Item 1, since the therapeutic light is appropriately controlled in accordance with the treatment effect (i.e., the progress of treatment), unnecessary therapeutic light will not be emitted to the subject.

### (Item 2)

The treatment support device as recited in the above-described Item 1,
wherein the control unit controls at least one of the irradiation time and the irradiation intensity of the therapeutic light such that the intensity of the fluorescence becomes equal to or less than a predetermined threshold.

According to the treatment support device as recited in the above-described Item 2, since the therapeutic light is controlled such that the intensity of fluorescence becomes equal to or less than the predetermined threshold, unnecessary therapeutic light irradiation to the subject is assuredly prevented.

### (Item 3)

The treatment support device as recited in the above-described Item 1, further comprising:
a storage unit configured to sequentially store the intensity of the fluorescence,
wherein the control unit
calculates an amount of change in the intensity of the fluorescence per predetermined time based on the intensity of the fluorescence stored in the storage unit, and
controls at least one of the irradiation time and the irradiation intensity of the therapeutic light such that the amount of change becomes equal to or less than a predetermined threshold.

According to the treatment support device as recited in the above-described Item 3, since the therapeutic light is controlled such that the amount of change in the intensity of the fluorescence becomes equal to or less than the predetermined threshold, unnecessary therapeutic light irradiation to the subject is assuredly prevented.

### (Item 4)

The treatment support device as recited in any one of the above-described Items 1 to 3, further comprising:
an imaging unit configured to acquire a fluorescence image by imaging the fluorescence generated from the fluorescent dye, when the therapeutic light is being emitted,
wherein the detection unit detects the intensity of the fluorescence based on a luminance value of the fluorescence image.

According to the treatment support device as recited in the above-described Item 4, the intensity of the fluorescence can be easily detected from the luminance value of the fluorescence image.

### (Item 5)

The treatment support device as recited in any one of the above-described Items 1 to 4,
wherein the therapeutic light is light having a wavelength of 600 nm to 750 nm.

According to the treatment support device as recited in the above-described Item 5, it is possible to assuredly excite the fluorescent dye.

### (Item 6)

A therapeutic light control method configured to control therapeutic light emitted toward a treatment site of a subject to whom a therapeutic agent containing a fluorescent dye has been administered and excite the fluorescent dye by means of the therapeutic light to perform treatment, the therapeutic light control method comprising the steps of:
emitting the therapeutic light;
controlling an irradiation time and irradiation intensity of the therapeutic light; and
detecting intensity of fluorescence generated from the fluorescent dye, when the therapeutic light is being emitted,
wherein the step of controlling the therapeutic light controls at least one of the irradiation time and the irradiation intensity of the therapeutic light based on the intensity of the fluorescence.

According to the therapeutic light control method of the above-described Item 6, the therapeutic light is appropriately controlled in accordance with the treatment effect (that is, the progress of treatment), so that unnecessary therapeutic light will not be emitted to the subj ect.

### (Item 7)

The therapeutic light control method as recited in the above-described Item 6,
wherein the step of controlling the therapeutic light controls at least one of the irradiation time and the irradiation intensity of the therapeutic light such that the intensity of fluorescence becomes equal to or less than a predetermined threshold.

According to the therapeutic light control method as recited in Item 7, since the therapeutic light is controlled such that the intensity of fluorescence becomes equal to or less than the predetermined threshold, unnecessary therapeutic light irradiation to the subject is assuredly prevented.

### (Item 8)

The therapeutic light control method as recited in the above-described Item 6, further comprising the step of:
sequentially storing the intensity of the fluorescence,
wherein the step of controlling the therapeutic light control
calculates an amount of change in the intensity of the fluorescence per predetermined time based on the intensity of the fluorescence stored in the storage unit, and
controls at least one of the irradiation time and the irradiation intensity of the therapeutic light such that the amount of change becomes equal to or less than a predetermined threshold.

According to the therapeutic light control method as recited in the above-described Item 8, since the therapeutic light is controlled such that the amount of change in the intensity of fluorescence becomes equal to or less than the predetermined threshold, unnecessary therapeutic light irradiation to the subject is assuredly prevented.

### (Item 9)

The therapeutic light control method as recited in any one of the above-described Items 6 to 8, further comprising the step of:
acquiring a fluorescence image by imaging the fluorescence generated from the fluorescent dye, when the therapeutic light is being emitted,
wherein the step of detecting the intensity of the fluorescence detects the intensity of the fluorescence based on a luminance value of the fluorescence image.

According to the therapeutic light control method as recited in the above-described Item 9, the intensity of the fluorescence can be easily detected from the luminance value of the fluorescence image.

### (Item 10)

The therapeutic light control method as recited in any one of the above-described Items 6 to 9,
wherein the therapeutic light is light having a wavelength of 600 nm to 750 nm.

According to the therapeutic light control method as recited in the above-described Item 10, the fluorescent dye can be assuredly excited.

### Description of Symbols

- 1:: Treatment support device
- 10:: Operation Unit
- 11:: Carriage
- 12:: Imaging unit
- 13:: Wheel
- 14:: Handle
- 15:: Image display unit
- 16:: Recess
- 17:: Control Unit
- 18:: Image forming unit
- 19:: Image composition unit
- 20:: Storage unit
- 24:: Light source unit
- 25:: Light source control unit
- 26:: Zoom lens
- 27:: Prism
- 28:: White light sensor
- 29:: Excitation light sensor
- 30:: Arm mechanism
- 31:: First arm member
- 32:: Second arm member
- 33:: Hinge portion
- 34:: Hinge portion
- 35:: Hinge portion
- 36:: Support
- 37:: Support portion
- 41:: Sub-arm
- 42:: Rotation shaft
- 43:: Support portion
- 181:: First imaging unit
- 182:: Second imaging unit
- 241:: First light source
- 242:: Second light source
- IM1:: Visible image
- IM2:: Fluorescence image
- IM3:: Composite image
- ST:: Subject

## Claims

1. A treatment support device configured to control therapeutic light emitted toward a treatment site of a subject to whom a therapeutic agent containing a fluorescent dye has been administered and excite the fluorescent dye by means of the therapeutic light to perform treatment, the treatment support device comprising:
a light source configured to emit the therapeutic light;
a control unit configured to control an irradiation time and irradiation intensity of the therapeutic light; and
a detection unit configured to detect intensity of fluorescence generated from the fluorescent dye, when the therapeutic light is being emitted,
wherein the control unit controls at least one of the irradiation time and the irradiation intensity of the therapeutic light based on the intensity of the fluorescence.

2. The treatment support device as recited in claim 1,
wherein the control unit controls at least one of the irradiation time and the irradiation intensity of the therapeutic light such that the intensity of the fluorescence becomes equal to or less than a predetermined threshold.

3. The treatment support device as recited in claim 1, further comprising:
a storage unit configured to sequentially store the intensity of the fluorescence,
wherein the control unit
calculates an amount of change in the intensity of the fluorescence per predetermined time based on the intensity of the fluorescence stored in the storage unit, and
controls at least one of the irradiation time and the irradiation intensity of the therapeutic light such that the amount of change becomes equal to or less than a predetermined threshold.

4. The treatment support device as recited in any one of claims 1 to 3, further comprising:
an imaging unit configured to acquire a fluorescence image by imaging the fluorescence generated from the fluorescent dye, when the therapeutic light is being emitted,
wherein the detection unit detects the intensity of the fluorescence based on a luminance value of the fluorescence image.

5. The treatment support device as recited in any one of claims 1 to 4,
wherein the therapeutic light is light having a wavelength of 600 nm to 750 nm.

6. A therapeutic light control method configured to control therapeutic light emitted toward a treatment site of a subject to whom a therapeutic agent containing a fluorescent dye has been administered and excite the fluorescent dye by means of the therapeutic light to perform treatment, the therapeutic light control method comprising the steps of:
emitting the therapeutic light;
controlling an irradiation time and irradiation intensity of the therapeutic light; and
detecting intensity of fluorescence generated from the fluorescent dye, when the therapeutic light is being emitted,
wherein the step of controlling the therapeutic light controls at least one of the irradiation time and the irradiation intensity of the therapeutic light based on the intensity of the fluorescence.

7. The therapeutic light control method as recited in claim 6,
wherein the step of controlling the therapeutic light controls at least one of the irradiation time and the irradiation intensity of the therapeutic light such that the intensity of fluorescence becomes equal to or less than a predetermined threshold.

8. The therapeutic light control method as recited in claim 6, further comprising the step of:
sequentially storing the intensity of the fluorescence,
wherein the step of controlling the therapeutic light control
calculates an amount of change in the intensity of the fluorescence per predetermined time based on the intensity of the fluorescence stored in the storage unit, and
controls at least one of the irradiation time and the irradiation intensity of the therapeutic light such that the amount of change becomes equal to or less than a predetermined threshold.

9. The therapeutic light control method as recited in any one of claims 6 to 8, further comprising the step of:
acquiring a fluorescence image by imaging the fluorescence generated from the fluorescent dye, when the therapeutic light is being emitted,
wherein the step of detecting the intensity of the fluorescence detects the intensity of the fluorescence based on a luminance value of the fluorescence image.

10. The therapeutic light control method as recited in any one of claims 6 to 9, wherein the therapeutic light is light having a wavelength of 600 nm to 750 nm.
